# EUROPEAN PATENT SPECIFICATION

(11) **EP 3 268 745 B1**
(45) Date of publication and mention of the grant of the patent: **01.07.2020**
(21) Application number: 16710797.8
(22) Date of filing: 11.03.2016
(51) Int. Cl.: G01N 33/68, A61K 38/00

(54) **ANTIBODIES AGAINST MODIFIED INSULIN FOR USE IN TREATMENT OF AND ASSAYS FOR DIABETES**
ANTIKÖRPER GEGEN MODIFIZIERTES INSULIN ZUR VERWENDUNG BEI DER BEHANDLUNG VON UND TESTS AUF DIABETES
ANTICORPS CONTRE L'INSULINE MODIFIÉE UTILES DANS LE TRAITEMENT DU DIABÈTE ET LE DÉPISTAGE DU DIABÈTE

(30) Priority: 13.03.2015 GB 201504297
(43) Date of publication of application: 17.01.2018
(73) Proprietor: Queen Mary University of London, London E1 4NS (GB)
(72) Inventor: NISSIM, Ahuva, London EC1M 6BQ (GB); POZZILLI, Paolo, London E1 2AT (GB); STROLLO, Rocky, 00128 Rome (IT)
(74) Representative: Lau, Sarah Jane
(86) International application number: PCT/GB2016/050678
(87) International publication number: WO 2016/146979

(56) References cited:
- PETER ACHENBACH ET AL: "Mature high-affinity immune responses to (pro)insulin anticipate the autoimmune cascade that leads to type 1 diabetes", JOURNAL OF CLINICAL INVESTIGATION, vol. 114, no. 4, 16 August 2004 (2004-08-16), pages 589-597, XP055278740, US ISSN: 0021-9738, DOI: 10.1172/JCI200421307
- SOFIA GUEDES ET AL: "Oxidative modifications in glycated insulin", ANALYTICAL AND BIOANALYTICAL CHEMISTRY, SPRINGER, BERLIN, DE, vol. 397, no. 5, 22 May 2010 (2010-05-22), pages 1985-1995, XP019839294, ISSN: 1618-2650
- STROLLO ROCKY ET AL: "Antibodies to post-translationally modified insulin in type 1 diabetes", DIABETOLOGIA, SPRINGER, BERLIN, DE, vol. 58, no. 12, 8 September 2015 (2015-09-08), pages 2851-2860, XP035578493, ISSN: 0012-186X, DOI: 10.1007/S00125-015-3746-X [retrieved on 2015-09-08]

## Description

### Field of the invention

The present invention relates to methods of diagnosing type 1 diabetes (T1D) in a subject comprising: testing a sample from the subject for the presence or absence of antibodies against oxidative post-translationally modified insulin; wherein the presence of antibodies against modified insulin in the sample is indicative of T1D in the subject. The present application further relates to uses of kits in these methods.

### Background to the invention

Type 1 diabetes (T1D) is a condition characterised by a lack of insulin as a result of destruction of insulin producing beta cells in the pancreas. Autoimmunity is believed to be the predominant effector mechanism which leads to the production of a number of autoantibodies that target the beta cells. T1D is primarily associated with children and adults under the age of 40. There is currently no effective therapy available for T1D.

Oxidative stress may be a critical player in the pathogenesis of type 1 diabetes (T1D). Hyperglycemia and high influx of metabolically active immune cells infiltrating the inflamed islets may result in formation of high levels of reactive oxidant species (ROS) within the β-cell microenvironment. The key ROS that are known to be produced are superoxide radical (O₂^{•-}), hydrogen peroxide (H₂O₂), hydroxyl radical (^{•}OH), hypochlorous acid (HOCl), nitric oxide (NO^{•}), peroxynitrite (ONOO-), and oxidants derived from glycation as a result of hyperglycemia. High levels of ROS may lead to oxidative posttranslational modification (oxPTM) of β-cell self-proteins and formation of neoepitopes, namely epitopes that were not previously presented to the immune system, and therefore escape immune tolerance and generate autoimmunity. The effect of ROS in inducing autoimmunity toward β-cell specific antigens remains largely unknown. The reason for the breakdown of tolerance to insulin in T1D has so far remained a mystery.

Over 30 years ago, evidence that insulin autoantibodies (IAA) are present in subjects with T1D was published in Science (Palmer et al., Science 222:1337, 1983). Achenbach et al., 2004, Journal of Clinical Investigation pages 589-597 also describes methods for detecting IAA to diagnose type 1 diabetes. IAA are established markers of T1D and may be used for the prediction of this disease. However, they are detected in only half of patients with newly-diagnosed T1D and in fewer patients diagnosed in adult age. Moreover, IAA titres are known to fluctuate during the progression of the disease. There is therefore a need for other biomarkers which can be used to diagnose T1D.

### Summary of the Invention

The present inventors have surprisingly found that immune response in type 1 diabetes (T1D) might be directed toward insulin modified by reactive oxygen species (ROS) rather than native insulin. The inventors have further shown that antibodies to modified insulin can be used as biomarkers for T1D. In particular, the presence of antibodies to modified insulin in a patient can be used to diagnose T1D. Accordingly, in a first aspect the invention provides a method of diagnosing type 1 diabetes (T1D) comprising:
testing a sample from a subject for the presence or absence of antibodies against oxidative post-translationally modified insulin;
wherein the presence of antibodies against modified insulin in the sample is indicative of T1D in the subject.

The subject matter for which protection is sought is as defined in the claims.

### Detailed description of the Invention

In a first aspect, the present invention relates to a method of diagnosing T1D. In other words, the invention relates to a method of determining whether a subject has T1D or, alternatively, a method of testing for T1D. The method of the invention can also be described as an assay.

The inventors have found that the method of the first aspect of the invention can be used to diagnose patients with new-onset T1D. The method is therefore typically carried out on a sample from a subject who is suspected of having T1D. The subject may, for example, be exhibiting one or more symptoms of T1D such as polydipsia (excessive thirst), polyuria (increased urination), polyphagia (increased appetite), weight loss, fatigue and other symptoms of diabetic ketoacidosis such as nausea, vomiting, abdominal pain, rapid deep sighing and progressive obtundation and loss of consciousness. The method is typically carried out on a sample from a subject who has not previously been treated with insulin or any other therapeutic agent for T1D, for example immunotherapeutics administered as vaccine (e.g. oral insulin, inhaled insulin, etc.) or immunosuppressive drugs.

The method of the first aspect of the invention involves testing a sample from a subject for the presence or absence of antibodies against modified insulin. "Modified insulin" as used herein refers to oxidative post-translationally modified insulin (oxPTM insulin). The terms "modified insulin" and "oxPTM insulin" are used interchangeably.

Insulin can be modified to oxPTM insulin by non-enzymatic glycation or by reactive oxygen species (ROS) or by reactions involving aldehydes or a combination thereof. Insulin modified by ROS is referred to herein as ROS-INS. Insulin modified by glycation is referred to as GLY-INS or Glyc INS.

Reactive oxygen species include, but are not limited to, for example, superoxide radical (O₂^{•-}), hydrogen peroxide (H₂O₂), lipid hydroperoxides (LOOH), lipid hydroperoxides in combination with transition metal ions (e.g. ferrous, ferric, cuprous or cupric salts), hydroxyl radical (^{•}OH), which is often generated in biological systems by hydrogen peroxide in combination with transition metal ions (e.g. ferrous, ferric, cuprous or cupric salts), hypochlorous acid (HOCl), hypobromous acid (HOBr), nitric oxide (NO^{•}), nitrogen dioxide (NO₂^{•}), and peroxynitrite (ONOO⁻). Some researchers in the field use terms such as "reactive nitrogen species" (e.g. NO^{•}) and "reactive chlorine species" (e.g. HOCl) to distinguish subgroups of ROS, but in this document we are using the term ROS in its broadest sense. Glycation is typically induced by glucose, ribose or by other sugars. Insulin can also be chemically modified by reactions involving aldehydes, for example malonaldehyde and/or 4-hydroxynonenal.

Typically, the modified insulin has been post-translationally modified by reactive oxygen species (ROS). In an embodiment of the invention, the ROS is typically hypochlorous acid (HOCI) or a hydroxyl radical (^{•}OH). Preferably the ROS is a hydroxyl radical (^{•}OH).

In some embodiments the modified insulin is oxidised insulin. Oxidised insulin is insulin which has been post-translationally modified by reactive oxygen species (ROS). In some embodiments the oxidised insulin is di-oxidised insulin or tri-oxidised insulin.

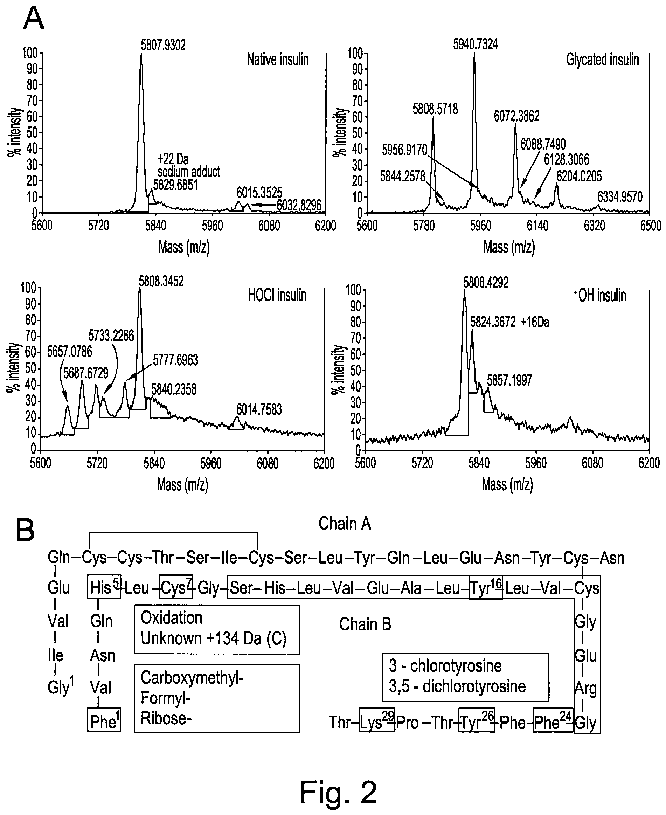

Insulin is a protein dimer of an A-chain and a B-chain (see Figure 2B). In some embodiments insulin chain B is modified by oxidation. Insulin B chain may be modified at at least one or more residues at positions 1 to 30. Insulin chain B may be modified at one or more of residues 1-13 (FVNQHLCGSHLVE), one or more of residues 14-21 (ALYLVCGE) and/or one or more of residues 21-30 (ERGFFYTPKT). In some embodiments insulin chain B is modified at one or more of residues 1-13 (FVNQHLCGSHLVE). In other embodiments insulin chain B is modified at one or more of residues 14-21 (ALYLVCGE). In other embodiments insulin chain B is modified at one or more of residues 21-30 (ERGFFYTPKT).

In some embodiments the modified insulin is modified by ROS at His5, Cys7, Tyr16, Phe24 and/or Tyr26 of the B chain. In some embodiments His5, Cys7 and/or Phe24 are modified by ^{•}OH and/or HOCI. In some embodiments Tyr16 and/or Tyr26 are modified by HOCI. In some embodiments His5, Cys7, Tyr16, Phe24 and/or Tyr26 of the B chain are oxidised.

In some embodiments the modified insulin is oxidised at His5, Cys7, Tyr16, Phe24 and/or Tyr26 of the B chain. In some embodiments the modified insulin is oxidised at one or more of His5, Cys7, Tyr16, Phe24 Tyr26 of the B chain. In some embodiments the modified insulin is oxidised at His5 of the B chain. In some embodiments the modified insulin is oxidised at Cys7 of the B chain. In some embodiments the modified insulin is oxidised at Tyr16 of the B chain. In some embodiments the modified insulin is oxidised at Phe24 of the B chain. In some embodiments the modified insulin is oxidised at Tyr26 of the B chain.

In some embodiments the modified insulin is modified by oxidation, chlorination, glycation, carboxymethlyation, formylation and/or ribosylation. The modified insulin may be oxidised insulin, chlorinated insulin, glycated insulin, carboxymethylated insulin, formylated insulin and/or ribosylated insulin.

The modified insulin may be suitably post-translationally modified by glycation. In some embodiments the glycation is ribosylation. Preferably, the glycation is induced by ribose, suitably D-ribose.

In some embodiments insulin chain B is modified by glycation. Insulin chain B may be modified at one or more of residues 1-13 (FVNQHLCGSHLVE), one or more of residues 14-21 (ALYLVCGE) and/or one or more of residues 21-30 (ERGFFYTPKT). In some embodiments insulin chain B is modified at one or more of residues 1-13 (FVNQHLCGSHLVE). In other embodiments insulin chain B is modified at one or more of residues 14-21 (ALYLVCGE). In other embodiments insulin chain B is modified at one or more of residues 21-30 (ERGFFYTPKT).

In some embodiments the modified insulin is glycated at Phe1 and/or Lys29 of the B chain.

In some embodiments the modified insulin is glycated at the N-terminus. The N-terminus may be the N-terminus of the B chain. The N-terminus of the B chain may be Phe1. In some embodiments the N-terminus is modified by ribosylation.

In some embodiments the modified insulin is glycated at a lysine residue of the B chain. The lysine residue of the B chain may be Lys29. In some embodiments the N-terminus is modified by ribosylation.

The term "modified insulin" as used herein also includes fragments of modified insulin, i.e. fragments of insulin that have been modified by ROS or by non-enzymatic glycation or any other method described herein. Such fragments modified by ROS are referred to herein as "ROS-fINS". The present invention therefore involves testing a sample from a subject for the presence or absence of antibodies against modified insulin or a fragment thereof. Fragments of modified insulin can, for example, be generated by cleavage of insulin with matrix metalloproteinases (MMPs) or ROS and can be further be modified by additional ROS.

The method involves testing a sample from a subject for the presence or absence of antibodies against modified insulin. The antibodies to be tested for are auto-antibodies against modified insulin that are present in the subject. As such the antibodies are typically of the immunoglobulin G (IgG) isotype or alternatively of the IgM or IgA isotype. In an embodiment the antibody is IgG.

The sample can be any sample from a subject that could contain auto-antibodies against modified insulin. Typically, the sample is a sample of blood, serum or plasma. In a preferred embodiment, the sample is a blood or serum sample.

The presence or absence of antibodies against modified insulin can be determined by any suitable method or assay. There is a wide range of different types of immunoassays available that can be used to measure autoantibodies in a sample. Typically, the presence or absence of such antibodies is determined using an assay based on antibody-antigen binding such as an ELISA assay or Western Blotting. In a typical ELISA assay, antigens are attached to a surface. Examples of fragments of modified insulin are shown in Table 1 below. Thus, in the present invention, modified insulin such as ROS-INS or fragments of modified insulin, for example fragments of insulin modified by ROS (ROS-fINS) are attached to a surface. A specific antibody (the primary antibody) is then applied over the surface and binds to the antigen. In this invention, the antibody is in the sample taken from the patient, so the sample is applied over the surface in this step of the method. A second antibody (the secondary antibody) is added, which binds to the primary antibody. The secondary antibody is linked to an enzyme, and then a substance containing the substrate of the enzyme is added. The subsequent reaction produces a detectable signal, typically a colour change in the substrate. The strength of the signal is indicative of the amount of the primary antibody. When the detectable signal is a colour change, a spectrometer is often used to give quantitative values for colour strength.

In one embodiment, an assay in accordance with the first aspect of the invention comprises: contacting a sample from a subject with modified insulin; adding a labelled antibody that binds to anti-modified insulin antibodies to form a complex; and detecting the formation of a complex between anti-modified insulin antibodies and labelled antibody; wherein the detection of said complex is indicative of T1D in the subject.

An antibody-antigen binding assay such as an ELISA assay for use in the present invention is carried out using modified insulin, for example ROS-INS, as a target for the antibodies in the sample taken from the subject. In such an assay, modified insulin can be prepared as the antibody target by chemical modification, for example using a ROS such as HOCI, ONOO-, ^{•}OH or using ribose, to produce modified insulin. In a suitable ELISA assay, ELISA plates are coated with modified insulin as bait to bind auto-antibodies from samples, for example blood or serum samples. Samples are then added to the ELISA plates, optionally with buffer added. Optionally, a non-reacting protein can then be added to block any surface of the ELISA plate that remains uncoated with modified insulin. An enzyme such as horseradish peroxidase can then be added. The enzyme is typically conjugated to an anti-human IgG antibody for binding to the antibodies from the sample. Finally, a substrate, for example a chromogenic substrate such as 3,3',5,5'-tetramethylbenzidine, is added. Activity can then be determined using a suitable method, for example by measuring the optical density (OD). Alternatively, fluorogenic or electrochemiluminescent reporters can be used as appropriate. Suitable ELISA assays for modified insulin are described in the Example herein.

Other suitable assays for modified insulin include: the multiplex assays available from Meso Scale Discovery which are based on the MULTI-ARRAY® technology and allow the assaying of many different antigens (for example insulin with different modifications) at the same time; multiplexed bead-based flow cytometry, for example the BD™ Cytometric Bead Array (CBA); and surface plasmon resonance (SPR) based systems, available for example from Biacore.

In some embodiments of the invention, the sample from the subject is compared to a control or a control sample. The control sample is typically a sample taken from a subject who is known not to be suffering from T1D, for example a healthy control subject. However, the control sample can also be, for example, from an individual presenting with symptoms of T1D but with no clinical evidence of T1D. The control sample can be from a patient with type 2 diabetes.

In further embodiments, reference positive and/or negative control samples are used. In other embodiments, native insulin (i.e. insulin that has not been modified) is used as a control. In other embodiments, native human serum albumin (HSA) or bovine serum albumin (BSA) or ROS modified BSA or HSA are used as control antigens. In another embodiment the control antigen is hen egg lysozyme (HEL).

In the method of the first aspect of the invention and the methods of the other aspects of the invention described herein, the presence or absence of antibodies against modified insulin can be determined by comparison to a control or a control sample, typically a control sample that is known not to contain antibodies against modified insulin. If the sample from the subject contains significantly higher levels of antibodies against modified insulin or fragments thereof than in the control sample, this confirms the presence of antibodies against modified insulin in the subject. Conversely, if the sample from the subject does not contain significantly higher levels of antibodies against modified insulin or fragments thereof than in the control sample, this confirms the absence of antibodies against modified insulin in the subject. The presence of antibodies against modified insulin or fragments thereof can be determined, for example, by finding a significant difference between the amounts of antibody in the sample versus a control sample. Statistical tests known in the art can be used, for example the Wilcoxon signed rank sum test or the Mann-Whitney test.

The subject is typically a human subject. However, the methods of the invention also find use in the field of veterinary medicine and can therefore be used to diagnose diabetes in animal subjects, typically mammalian subjects, for example companion animals such as cats and dogs, agricultural animals such as horses, cows, pigs and sheep, or other mammals such as mice, rats, rabbits or monkeys.

The methods of the invention are typically carried out on a sample that has previously been obtained from a subject. Thus, the taking of the sample does not typically form part of the methods of the invention and the methods of the invention are carried out on a sample that has been obtained from a subject. In some embodiments of the invention, however, the method also comprises taking the sample from the subject, for example by taking a blood sample (and optionally then preparing a serum sample from the blood sample).

The main autoantibodies associated with T1D include insulin autoantibodies (IAA), islet cell antibodies (ICA), glutamic acid decarboxylase autoantibodies (GADA), and insulinoma-associated-2 autoantibodies (IA-2A). IAA have become important diagnostic and prognostic tools in T1D, but the diagnostic sensitivity of IAA is not high (∼ 50%). ICA have been detected in approximately 70% new-onset T1D Caucasian patients. Following diagnosis, ICA frequency decreases, and fewer than 10% of patients still express ICA after 10 years. Approximately 60% onset cases of T1D express GADA. IA-2A and IA-2BA are observed in 60% or more of new-onset type 1 diabetes cases. Zinc transporter-8 (ZnT8) was recently identified as a novel autoantigen in T1D. Autoantibodies to ZnT8 (ZnT8a) have been detected in up to 80% of new-onset T1D.

The inventors have shown that auto-reactivity to insulin is in part due to neo-antigens resulting from oxidative post-translational modification (oxPTM) of insulin by the oxidants present in the β-cell microenvironment.

The present invention therefore provides a new method for improved diagnosis of T1D, because it can be used to diagnose patients who test negative for IAA, ICA, GADA, IA2A or ZnT8A.

Accordingly, in one embodiment, the invention provides a method of diagnosing T1D wherein a subject does not express detectable levels of IAA, ICA, GADA, IA2A and/or ZnT8A.

Tests to determine the presence or absence of autoantibodies are known in the art. These include radiobinding assay (RBA), radioimmunoassay (RIA), electrochemiluminescent assay (ECL-IAA) and ELISA.

The method of the first aspect of the invention gives a diagnosis of T1D in a subject. The method can therefore also be used in combination with a method of treating T1D.

Accordingly, the first aspect of the invention also extends to the medical uses as claimed.

Type 2 diabetes (T2D) is a metabolic disorder usually diagnosed in adults wherein the body produces insufficient amounts of insulin or develops insulin resistance resulting in elevated blood glucose levels. There is no cure for T2D but the condition can be managed through exercise and medication. There is usually no need to take exogenous insulin.

Examples of T2D medication include biguanides (such as metformin), enzyme inhibitors (such as angiotensin converting enzyme inhibitors (ACEI) and alpha-glucosidase inhibitors), Sulfonylureas (such as glyburide, glipizide, glimepiride, tolbutamide, chlorpropamide, acetohaxamide and tolazamide), meglitinides (such as repaglinide), thiazolidinediones (such as troglitazone, pioglitazone and rosiglitazone) and insulin and insulin analogues (such as lispro).

Although autoimmunity is not typically associated with T2D, up to 25% of people diagnosed with T2D have elevated levels of autoantibodies. Some people with T2D become unresponsive to diabetes medication and, as in the case of T1D patients, require insulin to control diabetes. This form of diabetes is known as latent autoimmune diabetes of adulthood (LADA) or type 1.5 diabetes because the condition starts off as type 2 diabetes before becoming type 1 diabetes.

In one embodiment, the T1D is LADA.

In a second aspect, the present disclosure provides a method of diagnosing latent autoimmune diabetes in adults (LADA) in a subject comprising:
testing a sample from the subject for the presence or absence of antibodies against modified insulin;
wherein the presence of antibodies against modified insulin in the sample is indicative of LADA in the subject.

The method of the second aspect is typically carried out on a sample from a subject who is known to have T2D. Accordingly, in one example, the subject has T2D or has been diagnosed with T2D. In another example, the subject is suspected of having T2D, for example because the subject exhibits one or more symptoms of T2D such as polydipsia (excessive thirst), polyuria (increased urination), polyphagia (increased appetite), weight loss and fatigue.

In an example of the second aspect, the sample is a sample of blood or serum.

In another example the insulin is post-translationally modified by ROS. The ROS is typically hypochlorous acid (HOCI) or a hydroxyl radical (^{•}OH). Preferably the ROS is a hydroxyl radical (^{•}OH).

In another example the insulin is modified by glycation. Preferably, the glycation is induced by ribose.

The method of the second aspect gives a diagnosis of LADA in a subject. The method can therefore also be used in combination with a method of treating LADA.

Accordingly, the second aspect also extends to a method of treating latent autoimmune diabetes in adults (LADA) in a subject in need thereof, comprising:
testing a sample from the subject for the presence or absence of antibodies against modified insulin;
identifying the presence of antibodies against modified insulin in the sample; and
administering a therapeutic agent for LADA to the subject.

The therapeutic agent is typically insulin, an immunotherapeutic administered as vaccine (e.g. oral insulin, inhaled insulin, etc.) or an immunosuppressive drug. Most typically, the therapeutic agent is insulin.

This example also extends to a therapeutic agent for latent autoimmune diabetes in adults (LADA) for use in to a method of treating LADA in a subject in need thereof, wherein the method comprises:
testing a sample from the subject for the presence or absence of antibodies against modified insulin;
identifying the presence of antibodies against modified insulin in the sample; and
administering a therapeutic agent for LADA to the subject.

This example also extends to use of a therapeutic agent for latent autoimmune diabetes in adults (LADA) in the manufacture of a medicament for the treatment of LADA in a subject in need thereof by a method comprising:
testing a sample from the subject for the presence or absence of antibodies against modified insulin;
identifying the presence of antibodies against modified insulin in the sample; and administering a therapeutic agent for LADA to the subject.

In a third aspect, the disclosure provides a method of determining the therapeutic efficacy of a therapeutic agent to treat a disease associated with oxPTM insulin.

In an example of the third aspect, the disease is T1D. In another example the disease is LADA.

According to the third aspect, the therapeutic effectiveness of a therapeutic agent to treat T1D or LADA is determined by:
i) determining the level of antibodies against modified insulin in a first sample that has been obtained from a subject prior to administering the therapeutic agent to the subject;
ii) determining the level of antibodies against modified insulin in a second sample that has been obtained from the subject after administering the therapeutic agent to the subject; and
iii) comparing the levels of antibodies against modified insulin between the first and second samples;
wherein a decrease in the level of antibodies against modified insulin in the second sample when compared to the first sample is indicative of therapeutic effectiveness of the therapeutic agent.

In some examples, the method also includes the steps of obtaining a first and a second sample from the subject and administering the therapeutic agent to the subject. Accordingly, in one example of the third aspect, the therapeutic effectiveness of a therapeutic agent to treat T1D or LADA is determined by:
i) obtaining a first sample from a subject;
ii) determining the level of antibodies against modified insulin in the first sample;
iii) administering the therapeutic agent to the subject;
iv) obtaining a second sample from the subject;
v) determining the level of antibodies against modified insulin in the second sample; and
vi) comparing the levels of antibodies against modified insulin between the first and second samples;
wherein a decrease in the level of antibodies against modified insulin in the second sample when compared to the first sample is indicative of therapeutic effectiveness of the therapeutic agent.

As used herein, the term "therapeutic agent" refers to a compound that provides a desired biological or pharmacological effect when administered to a subject. Examples of therapeutic agents to treat T1D or LADA include but are not limited to insulin, immunotherapeutics administered as vaccine (e.g. oral insulin, inhaled insulin, etc.) or immunosuppressive drugs.

The method of the third aspect is for identifying whether a subject responds to such a diabetes medication (i.e. a therapeutic agent to treat T1D or LADA). By "respond to a diabetes medication" is meant respond to treatment with a diabetes medication, in other words reduce the severity of the symptoms of T1D in the patient and improve glucose control as measured by blood glucose, HbA1c and C-peptide. Response to treatment with a diabetes medication can be determined by assessing blood glucose control before and after treatment with the diabetes medication.

The method of the third aspect is typically carried out on a sample from a subject that has previously been treated or is currently being treated with one or more diabetes medications. In this aspect, the present disclosure relates to a method of monitoring whether a patient is responding to therapy with one or more diabetes medications (i.e. a therapeutic agent to treat T1D or LADA).

The method of the third aspect gives an indication of whether a patient responds to a therapeutic agent to treat T1D or LADA. The third aspect therefore also encompasses a method which comprises a further step of treating a patient with a therapeutic agent to treat T1D or LADA, i.e. administering a therapeutic agent for T1D or LADA to the subject. The therapeutic agent is typically insulin, an immunotherapeutic administered as vaccine or an immunosuppressive drug.

In all aspects, the method of treatment can be of a human or animal subject and this extends equally to uses in both human and veterinary medicine. The therapeutic agent to treat T1D or LADA is preferably administered to a subject in a "therapeutically effective amount", this being sufficient to show benefit to the subject and/or to ameliorate, eliminate or prevent one or more symptoms of T1D or LADA. As used herein, "treatment" includes any regime that can benefit a human or a non-human animal, preferably a mammal. The treatment may be in respect of an existing condition or may be prophylactic (preventative treatment). The treatment is typically administered to a subject or patient "in need thereof", i.e. a subject suffering from T1D or LADA.

The therapeutic agent to treat T1D or LADA can be administered to the subject by any appropriate route, for example by oral (including buccal and sublingual), nasal, topical (including transdermal) or parenteral (including subcutaneous, intramuscular, intravenous, intraperitoneal and intradermal) administration, although when the therapeutic agent to treat T1D or LADA is insulin it will typically be administered to the subject by intravenous administration. The therapeutic agent to treat T1D or LADA can be formulated using methods known in the art of pharmacy, for example by admixing the therapeutic agent with carrier(s) or excipient(s) under sterile conditions to form a pharmaceutical composition. Accordingly, in one example the subject is administered a pharmaceutical composition comprising a therapeutic agent to treat T1D or LADA and one or more carriers and/or excipients.

The therapeutic agent to treat T1D or LADA can also be administered in combination with one or more other therapeutically active agents. Accordingly, the pharmaceutical composition for use in accordance with this example may also comprise one or more other therapeutically active agents in addition to the therapeutic agent to treat T1D or LADA.

Dosages of the therapeutic agent to treat T1D or LADA and/or pharmaceutical composition can vary between wide limits, depending for example on the particular therapeutic agent used, the age and disease stage of the patient, and a physician will ultimately determine appropriate dosages to be used.

The dosage can be repeated as often as appropriate. If side effects develop, the amount and/or frequency of the dosage can be reduced, in accordance with normal clinical practice.

In a fourth aspect the invention provides the use of a kit for diagnosing T1D or LADA in a subject. The kit comprises one or more reagents for determining the presence of antibodies against modified insulin. The kit is packaged and labelled for example, in a box or container which includes the necessary elements of the kit, and directions and instructions on the use of such diagnostic kit.

In some instances the uses of the kits of the present invention may be useful in predicting a subject's risk of developing T1D or LADA.

The present invention will now be further described by way of reference to the following Examples which are present for the purposes of illustration only. In the Examples, reference is made to a number of Figures in which:

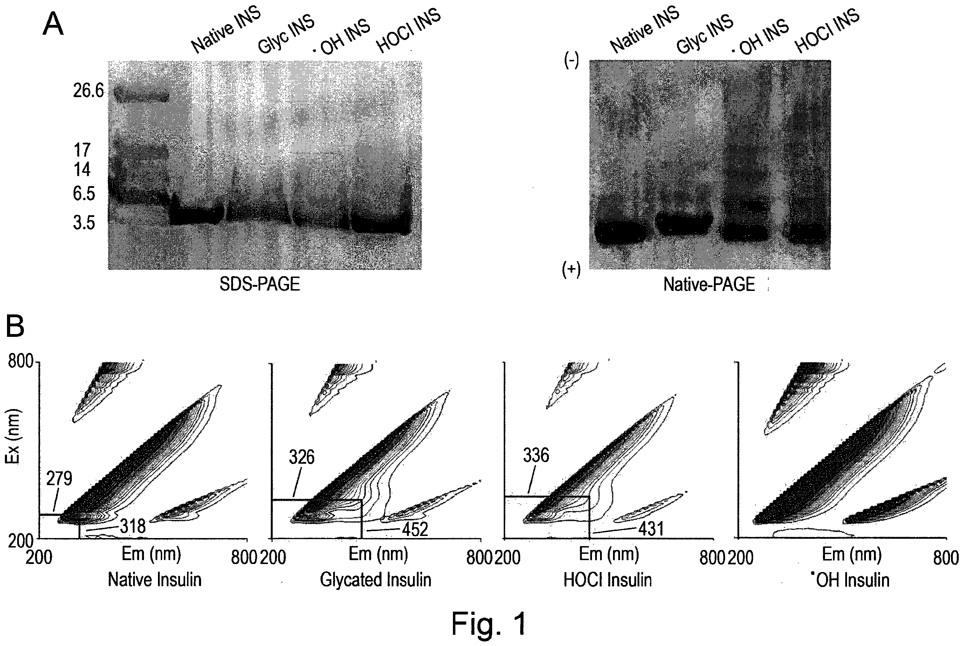

FIG. 1. Analysis of native and modified insulin by gel electrophoresis and 3D fluorescence. **A:** Sodium dodecyl sulfate (SDS) and native polyacrylamide gel electrophoresis (PAGE) analysis of insulin (INS) modified by reactive oxidants: glycation by ribose (Glyc INS), ^{•}OH (^{•}OH -INS) and HOCI (HOCI-INS). The position of the molecular weight markers (in kDa) is shown on the left panel. The native PAGE showed a clear reduction in mobility of the insulin after glycation by ribose as the appearance of two bands with slower mobility. Exposure of insulin to the HOCI and ^{•}OH -generating systems induced the appearance of additional and slower mobility bands, and a smear of protein through the entire line suggesting fragmentation. **B:** Three-dimensional fluorescence profile of native insulin and insulin modified by glycation, HOCI and ^{•}OH. Modification of insulin with HOCI or ribose resulted in a substantial shift in the Emmax and Exmax wavelength indicated by vertical and horizontal lines and associated numbers in the figure. Modification with ^{•}OH resulted in loss of the native fluorescence and increased in light scattering suggesting aggregation of the native molecules.
FIG. 2. Analysis of native and modified insulin by mass spectrometry. **A:** MALDI-TOF analysis of native and ROS-modified insulin. Native insulin showed a peak at m/z 5,807.65 Da; after glycation by ribose, four additional peaks appeared with mass differences of 132 Da and corresponding to the mono-, di-, three- and tetra-glycated insulin forms. Exposure to HOCI induced the development of additional peaks at lower m/z respect to the native form, indicating the formation of degradation products induced by the reaction with the oxidant. A peak at m/z 5,824 (+16) was shown for insulin modified by ^{•}OH; the mass difference between native insulin and the ^{•}OH -insulin was 16Da, which corresponds to one oxidation. **B:** MS/MS analyses of ROS modified insulin showed that the main oxidative changes induced by ^{•}OH and HOCI involved His5, Cys7 and Phe24 in the B-chain (upper, left hand box "Oxidation Unknown +134 Da (C)"). In addition treatment with HOCI induced the generation of chlorine species on Tyr16 and Tyr26 (right hand box, "3-chlorotyrosine, 3,5-dichlorotyrosine"), while the main modifications by ribose involved N-terminal Phe1 and Lys29 in the B-chain (lower, left hand box "Carboxymethyl-, Formyl-, Ribose-"). The shaded area indicates the immunodominant epitope B9:23.

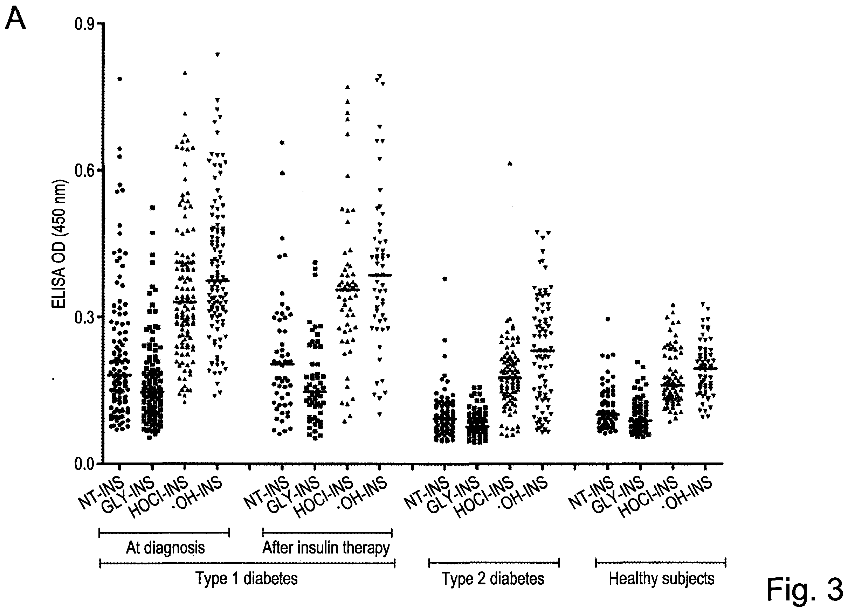

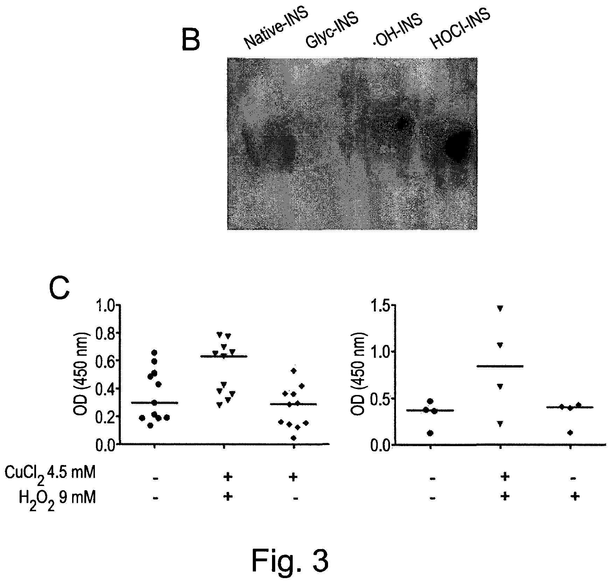

FIG. 3. Binding of T1D serum to native and modified insulin **A:** ELISA binding to insulin by serum samples from patients with T1D or T2D and healthy controls. Reactivity to native (NT-INS) and to ROS-insulin (ROS-INS) modified by ribose (GLY-INS), HOCI (HOCI-INS) and ^{•}OH (^{•}OH -INS) were significantly higher in T1D compared with T2D and healthy controls (p<0.001). Hence binding to ROS-INS was significantly higher than to native-INS in T1D (p<0.0001); Binding to ROS-INS remained higher than native also after insulin therapy (P<0.001). Binding at diagnosis and after six-twelve months after diagnosis is showed. **B:** Binding to native and ROS-INS as detected by western blot. Binding to native insulin and a stronger intensity binding to a smear of smaller mobility fragment of ^{•}OH -INS and HOCI-INS was observed. No binding to GLY-INS was seen. **C:** Binding of serum to hydroxyl radical-modified insulin by ELISA. To exclude that serum binding was due to insulin hexamerization by the bivalent metal used in the hydroxyl radical generating system, insulin was exposed to CuCl₂ (4.5 mM) in absence of H₂O₂ and compared with exposure to H₂O₂ alone (9 mM) or to the hydroxyl radical generating system by Fenton reaction (CuCl₂+ H₂O₂). Incubation of insulin with Cu₂⁺ increased reactivity only in presence of H₂O₂, confirming the involvement of Fenton reaction to the increased ELISA binding.

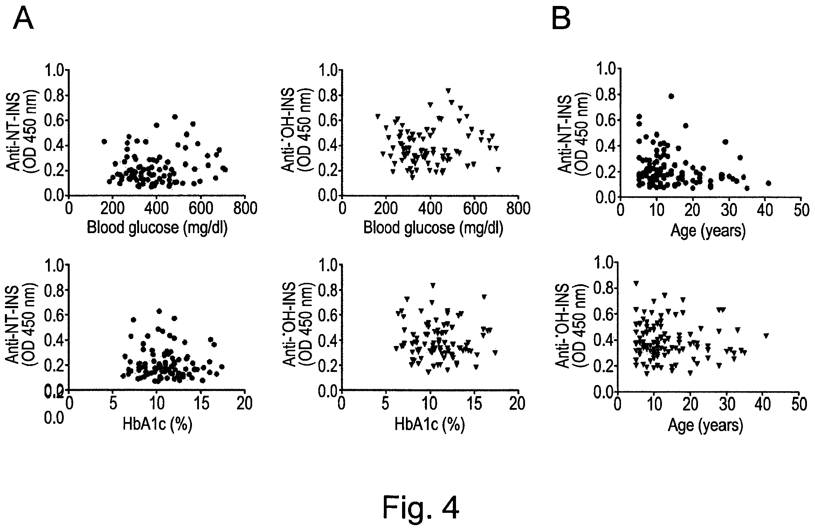

FIG. 4. Relationship between native and modified insulin with glucose control and age. **A:** Binding of samples from type 1 diabetic individuals to native and modified insulin was not related to indices of glucose control, such as fasting plasma glucose and HbA1c (0.07<ρ<0.16, P>0.132). **B:** Native and glycated INS were inversely correlated with age (ρ= - 0.20, P=0.031 and ρ= -0.21, P=0.029, respectively); no age specificity was found for the two other ROS-INS (-0.14<ρ<-0.08, P>0.130).

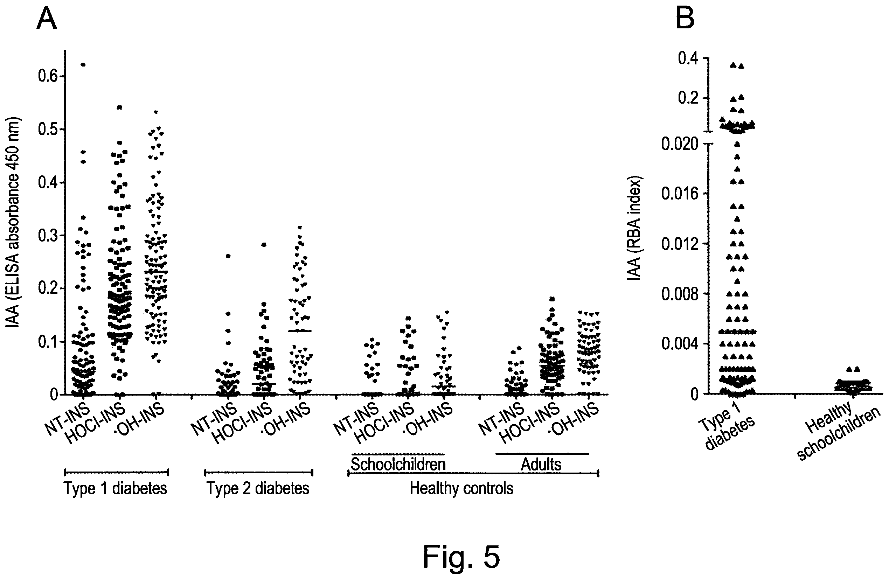

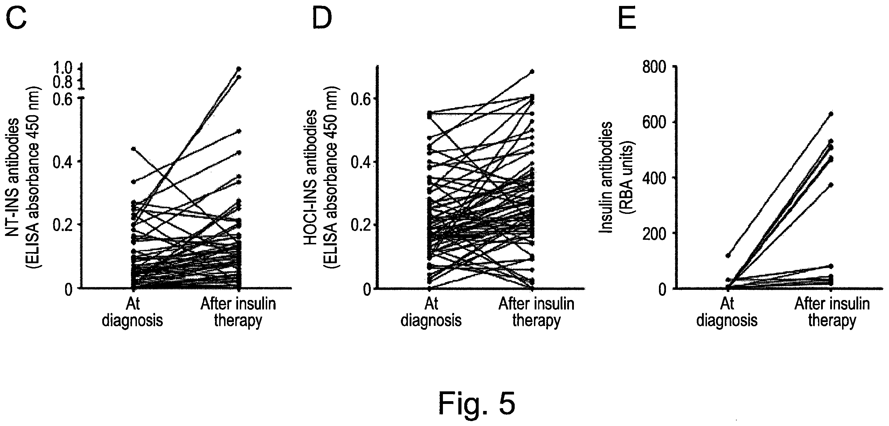

FIG. 5. Binding of type 1 diabetes serum to native insulin (NT-INS) and oxPTM-INS. **(A)** ELISA binding to insulin by serum samples from patients with type 1 diabetes, patients with type 2 diabetes and healthy controls. Reactivity to NT-INS and oxPTM-INS was significantly higher in samples from those with type 1 diabetes compared with type 2 diabetes and healthy controls (*p*<0.001). Binding to oxPTM-INS was significantly higher than to NT-INS in samples from participants with type 1 diabetes (*p*<0.0001). **(B)** Levels of IAA were assessed by RBA in the same group of patients with type 1 diabetes and healthy schoolchildren. **(C-E)** Longitudinal changes in antibody binding to **(C)** NT-INS (*p*<0.0001) and **(D)** HOCI-INS (*p*=0.007) by ELISA and **(E)** insulin antibodies assessed by RBA (*p*<0.0001), before and after insulin therapy

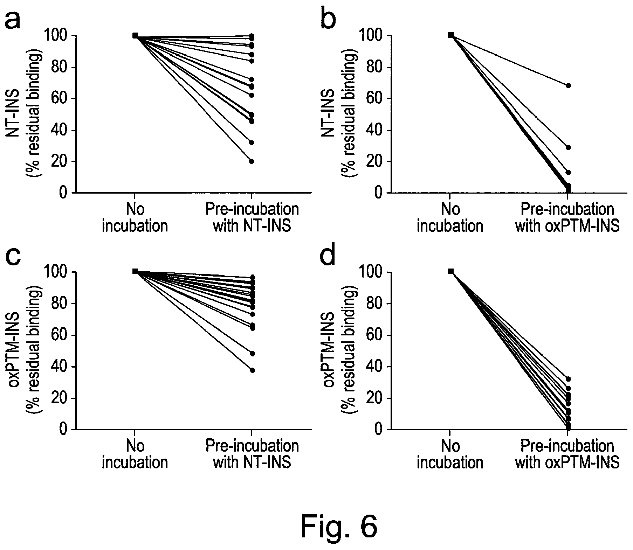

FIG. 6. Serum binding specificity to oxPTM-INS. Pre-incubation of type 1 diabetes serum samples with oxPTM-INS, but not with native insulin (NT-INS), strongly inhibited binding to oxPTM-INS, indicating the presence of antigen-binding sites specific to oxPTM-INS. Data are shown for insulin modified by ^{•}OH, as example of oxPTM-INS

### Examples

### Example 1 - Modification of insulin by ROS

The inventors tested a cohort of patients with T1D early in the onset for their reactivity to both native insulin and insulin exposed to reactive oxidants such as hydroxyl radicals (^{•}OH), Hypoclorous acid (HOCI), and ribose. The structural modification was then analysed by polyacrylamide gel electrophoresis (PAGE), 3D fluorescence and mass spectrometry (MS). In SDS-PAGE analysis ROS modified insulin (ROS-INS) and native insulin migrated to the region of 5kDa with no significant difference in mobility, although a smear above the major 5kDa band was observed for the ROS-INS (Fig. 1A, SDS-PAGE). In non-denaturing PAGE, differences between native and modified insulin emerged. After glycation by ribose a clear shift in the position of the insulin band plus two additional bands all with slower mobility appeared. Exposure of Insulin to the ^{•}OH -generating system and HOCI induced the appearance of additional and slower mobility bands, in conjunction with a smear of protein through the entire line suggesting fragmentation. (Fig. 1A, Native-PAGE).

To determine the effect of ROS modifications on the structural changes of insulin the inventors then performed a 3D fluorescence profile study of both native and ROS-INS (Fig. 1B). Some intrinsic fluorescence was detectable in the native insulin with a maximum excitation Exmax=279 nm and maximum emission Emmax=318 nm, which can be attributed to tyrosine residues. Modification of insulin with ribose resulted in a substantial shift in Exmax and Emmax to 326 and 452 nm, respectively, and after modification with HOCI to Exmax=336 and Emmax=431 nm, which implies changes in the insulin structure. Modification with ^{•}OH resulted in loss of fluorescence and increase in light scattering suggesting aggregation of the native molecules.

Native and modified insulin were then analysed by high-resolution mass spectrometry (MS). Native insulin showed a peak at m/z 5,807.65Da corresponding to the molecular mass of insulin. After glycation by ribose, four additional peaks appeared with mass differences of 132Da indicating the addition of ribose molecules to insulin and corresponding to the mono-, di-, tri- and tetraglycated insulin forms. Exposure to HOCI induced the development of additional peaks at lower m/z respect to the native form, indicating the formation of degradation products induced by the reaction with the oxidant. A peak at m/z 5,824 (+16) was shown for insulin modified by ^{•}OH; the mass difference between native insulin and the ^{•}OH -insulin was 16Da, which corresponds to one oxidation (Fig. 2A). MS/MS analysis suggested that the main oxidative changes involved Phe1 and Lys29 (glycation), His5, Cys7, Phe24 (^{•}OH and HOCI) and Tyr16 and Tyr26, (HOCI), in the B-chain (Fig. 2B, Table 1). Our observation correlate with previous study showing that glycation of insulin involves the N-terminal Phe1and Lys29 in the B-chain. Similarly, the oxidation in a more central correlate with previous report implicating Try16 and 26, Phe24 and Cys19 as the preferred site of changes.

**Table 1: MS/MS analysis of insulin B-Chain. The table shows the main oxidative post translational modifications (PTMs) involving the peptides B:1-13 (FVNQHLCGSHLVE), B:14-21 (ALYLVCGE) and B:21-30 (ERGFFYTPKT). No changes were detected in the A-chain. Underlined residues indicate the position of the relevant oxidative PTM.**

| | **MH+ found** | **Sequence** |
|---|---|---|
| **Native INS** | 1482.7268 | -.FVNQHLCGSHLVE.A |
| | 1514.6863 | -.FVNQHLCGSHLVE.A + Dioxidation (C) |
| | 867.43 | ALYLVCGE |
| | 1116.5885 | E.RGFFYTPKT.G |
| | | |
| **Glycated INS** | 1482.6830 | -.FVNQHLCGSHLVE.A |
| | 1510.6870 | -.FVNQHLCGSHLVE.A + Formyl (N-term) |
| | 1540.68 | -.FVNQHLCGSHLVE.A + Carboxymethyl (N-term) |
| | 1614.73 | -.FVNQHLCGSHLVE.A + D-Ribose (N-term) |
| | 1116.5745 | E.RGFFYTPKT.G |
| | 1144.5599 | E.RGFFYTPKT.R + Formyl (K) |
| | 1174.5616 | E.RGFFYTPKT.R + Carboxymethyl (K) |
| | 1248.61 | E.RGFFYTPKT.R + D-Ribose |
| | 1194.5848 | E.RGFFYTPKT.R + D-Ribose - 3H₂O |
| | | |
| **^{•}OH -INS** | 1482.6886 | -.FVNQHLCGSHLVE.A |
| | 1498.6687 | -.FVNQHLCGSHLVE.A + Oxidation (HW) |
| | 1514.6658 | -.FVNQHLCGSHLVE.A + Oxidation (C); Oxidation (HW) |
| | 1514.6863 | -.FVNQHLCGSHLVE.A + Dioxidation (C) |
| | 1530.68 | -.FVNQHLCGSHLVE.A + Trioxidation (C) |
| | 1116.5701 | E.RGFFYTPKT.G |
| | 1132.5 | RGFFYTPKT + Oxidation (F) |
| | | |
| **HOCI-INS** | 1482.7009 | -.FVNQHLCGSHLVE.A |
| | 1530.6876 | -.FVNQHLCGSHLVE.A + Oxidation (C); Oxidation (F); Oxidation (HW) |
| | 1530.6876 | -.FVNQHLCGSHLVE.A + Trioxidation (C) |
| | 867.43 | ALYLVCGE |
| | 901.39 | ALYLVCGE +3-ChloroTyrosine |
| | 1116.5859 | E.RGFFYTPKT.G |
| | 1150.5411 | RGFFYTPKT +3-ChloroTyrosine |
| | 1184.4957 | RGFFYTPKT +3,5-DiChloroTyrosine |

### Example 2 - Antibody binding to native and ROS-INS in sera from subjects with T1D

Native and ROS-INS were used as targets in ELISA to compare antibody binding to native versus ROS-INS in sera from subjects with T1D. As controls, samples from healthy subjects and patients with type 2 diabetes (T2D) were used. To avoid interference with antibodies against exogenous insulin, patients with T1D, mean age 13.90±0.77 years, were all at diagnosis before insulin therapy was started (Table 2). Reactivity to native and ROS-INS was detected in 34% and 79% of T1D patients, respectively, and with a mean ± SD OD of 0.22 ± 0.13, 0.35 ± 0.14, and 0.39 ± 0.14 for binding to native insulin, HOCI-INS, and ^{•}OH -INS, respectively (Fig. 3A). Binding to HOCI- and ^{•}OH -INS was significantly higher in comparison with binding to native insulin (P<0.0001). Paradoxically, glycated insulin showed a significant reduction in binding compared with native insulin (P=0.018, 0.22 ± 0.13 vs 0.16 ± 0.08). Binding of HOCI- and ^{•}OH -INS was higher in T1D sera than in T2D and healthy subjects (P<0.0001). In T2D we observed 4%, 0%, 3% and 28%, binders to native insulin, glycated-INS, HOCI-INS and ^{•}OH -INS, respectively. In healthy individuals, 3% binders were observed to both native and ROS-INS (Table 3). The sensitivity and specificity of auto-antibody binding to HOCI-INS in patients with T1D were 79.31% and 97.14%, respectively, compared with healthy controls and 65.52% and 96.34%, respectively compared to T2D. However, reactivity to ^{•}OH -INS was less specific in patients with T1D compared with T2D patients, with sensitivity and specificity of 76.72% and 71.95%, respectively. Interestingly, treatment with exogenous insulin induced no significant differences in the longitudinal antibody binding to ROS-INS (P>0.092) while binding to native insulin increased slightly (OD 0.188±0.11 vs. 0.218±0.12; P=0.011), possibly elicit from immune response to the exogenous insulin injected. Overall, during a timeframe of 6-12 months, binding to ROS-INS remained significantly higher than to native-INS (P<0.0001; Fig. 3A).

To eliminate the possibility that binding is to modified amino acid rather to structural neoepitope induced by oxPTM, by testing the reactivity to control antigen (Table 3). Hence, we excluded the possibility that binding was due to insulin hexamerization by the bivalent metal used in the ^{•}OH - generating system, by exposing insulin to Cu₂⁺ (Figure 3C). Therefore, the anti-modified insulin immune recognition was specific and was directed against insulin neo-epitopes induced by the oxPTM. Western blot analysis was then performed on a range of serum samples that exhibited the strongest binding in ELISA. For native insulin, binding to a fragment in the region of 5kDa was evident, but this was lost after glycations. Binding to ^{•}OH -INS and HOCI-INS was stronger and directed toward a diffused fragment that had slower mobility than the native-INS (Fig. 3B). Western blot analyses confirmed the ELISA results and suggested the presence of intrinsic specificities of the neo-epitopes induced by the different ROS-generating systems. Serum sample from healthy control did not bind to any of the insulin format (data not shown)

**Table 2: Clinical and biochemical features of the studied populations. Gender, age and metabolic parameters of type 1 diabetes mellitus (T1D), type 2 diabetes mellitus (T2D) and healthy controls. Data are mean ± standard error.**

| **Characteristic** | **T1D** | **T2D** | **Healthy subjects** |
|---|---|---|---|
| | **(N=116)** | **(N=82)** | **(N=68)** |
| Aqe, years | 13.90±0.77 | 57.20±1.76 | 37.96±1.41 |
| Males:Females ratio | 0.85 | 0.81 | 0.66 |
| Blood glucose, mg/dl | 388.11±13.58 | 147.54±5.06 | |
| HbA1c, % | 10.98±0.27 | 6.89±0.19 | |
| BMI, kg/m² | 18.67±0.40 | 30.63±5.56 | |

**Table 3: Binding to native and ROS-INS. ELISA OD levels that were above the 97.5th percentile of the healthy subjects were defined as elevated. Binding to native and insulin modified by reactive oxidants (ROS-INS) was higher in type 1 diabetes (T1D) compared with control type 2 diabetes (T2D) and healthy individuals (P<0.001). In T2D, Binding to insulin modified by ^{•}OH (^{•}OH-IAA) was higher compared with healthy subjects but lower than T1D. To eliminate the possibility that binding to ROS-insulin in the sera could be attributed to specific binding to a given modified amino acid, we tested a non-relevant native and ROS modified, hen egg lysozyme (HEL), as target antigen for binding. In all groups, antibodies to glycated and ^{•}OH -modified HEL were lower than native HEL antibodies, while only binding to HOCI-modified HEL tended to be higher than to native HEL.**

| | **T1D** | **T2D** | **Healthy subjects** |
|---|---|---|---|
| | **(N=116)** | **(N=82)** | **(N=68)** |
| Autoantibodies to INS | | | |
| Native-INS | 40 (34%) | 3 (4%) | 2 (3%) |
| Glycated-INS | 38 (33%) | 0 (0%) | 2 (3%) |
| HOCI-INS | 76 (66%) | 3 (4%) | 2 (3%) |
| ^{•}OH -INS | 89 (77%) | 23 (28%) | 2 (3%) |
| Autoantibodies to HEL | | | |
| Native-HEL | 18(16%) | 2 (2%) | 2 (3%) |
| Glycated-HEL | 14 (12%) | 2 (2%) | 2 (3%) |
| HOCI-HEL | 45 (39%) | 12 (15%) | 2 (3%) |
| ^{•}OH -HEL | 9 (8%) | 0 (0%) | 2 (3%) |

### Example 3 - Relationship between native and ROS-INS with glucose control and age

As shown for other ROS antigens (Strollo et al, Diabetologica, Mar 2013, Vay et al Diabetologica Nov 2000), the levels of both anti-native-INS and ROS-INS at diagnosis were not correlated with blood glucose or HbA1c (0.07<ρ<0.16, P>0.132; Fig. 4A). Hence, as demonstrated by Vardi et al (Diabetes Care Oct 1988) anti-native-INS was inversely correlated with age (ρ= -0.20, P=0.031), but no age correlation was found for anti-ROS-INS (-0.14<ρ<-0.08, P>0.130, Fig. 4B). Interestingly, although limited by the small sample size, the inventors did not find any clear HLA association with DRB1*03 or DRB1*04 known to be associated with T1D (Table 4). This result is opposite to the inventors' previous study where HLA specificity was associated with anti-ROS-CII (Strollo et al, Mar 2013).

**Table 4: HLA-DRB1 and anti-ROS-INS reactivity. Anti-ROS-INS are not related to HLA-DRB1 genotypes in type 1 diabetes. Complete DR typing was available for 90 type 1 diabetes subjects. DRB1*X does not equal to DRB1*04, DRB1*03.**

| **HLA-DR type** | **Native INS autoantibodies** | | | **ROS-INS autoantibodies** | | |
|---|---|---|---|---|---|---|
| | **N positive** | **Odds ratio (95% CI)** | ***P* value** | **N positive** | **Odds ratio (95% CI)** | ***P* value** |
| *ORB1***03*/not *DRB1***04* (N=22) | 7 | 1.287 (0.471, 3.799) | 0.636 | 18 | 1.950 (0.695, 5.611) | 0.211 |
| *ORB1***04*/*not DRB1***03* (N=28) | 5 | 0.531 (0.175, 1.615) | 0.262 | 17 | 0.792 (0.314, 1.992) | 0.620 |
| *ORB1***03*/*DRB1***04* (N=23) | 4 | 0.532 (0.160, 1.769) | 0.300 | 11 | 0.390 (0.148, 1.030) | 0.057 |
| *ORB1***X*/*ORB1***X* (N=17) | 7 | 2.494 (0.819, 7.595) | 0.108 | 13 | 2.022 (0.599, 6.822) | 0.256 |

### Example 4 - Antibody binding to ROS-INS in patients with T1D and control individuals

Antibody binding to ROS-INS in patients with type 1 diabetes and control individuals Native insulin and ROS-INS were used as targets in ELISA to assess antibody binding to native insulin vs ROS-INS in sera from individuals with type 1 diabetes. As controls, samples from a group of healthy schoolchildren and a group of adults were used. To avoid interference with antibodies against exogenous insulin, samples from patients with type 1 diabetes (mean ± SE age 13.90 ± 0.77 years) were taken at diagnosis, before the patients started insulin therapy (Table 4). Binding to insulin modified by HOCI (HOCI-INS) and ^{•}OH-INS was significantly higher than binding to native insulin. Reactivity to native insulin and ROS-INS was detected in 30% and 84%, respectively, of patients with type 1 diabetes, with median absorbances of 0.043±0.012, 0.183±0.012 and 0.231±0.013 for binding to native insulin, HOCI-INS and ^{•}OH-INS, respectively (p<0.0001, Fig. 5a). Binding to glycated insulin was significantly lower compared with native insulin (absorbance 0.007 ± 0.006, p<0.0001, data not shown). Antibodies to the control antigens HEL and oxPTM-HEL were present in only 5% and 3.5% participants with type 1 diabetes, respectively (data not shown). Binding of HOCI-INS and ^{•}OH-INS was higher in sera from participants with type 1 diabetes than in that from participants with type 2 diabetes and healthy individuals (p<0.0001). The sensitivity and specificity of ROS-INS reactivity in patients with type 1 diabetes were 84% and 99%, respectively, compared with healthy schoolchildren, and 66% and 99%, respectively, compared with patients with type 2 diabetes.

To exclude the possibility that serum reactivity was due to metabolic alteration at the time of the clinical onset of diabetes, binding in 69 patients with type 1 diabetes was evaluated longitudinally after insulin treatment (Fig. 5c-e). Insulin therapy induced a slight increase in binding to native insulin (median absorbance 0.040 ± 0.014 vs 0.083 ± 0.026; p<0.0001) and HOCI-INS (median absorbance 0.187 ± 0.019 vs 0.262 ± 0.024; p=0.007), but not to ^{•}OH-INS (data not shown). Overall, binding to ROS-INS remained significantly higher than to native insulin (p<0.0001). Intra-assay CV of triplicates was <8% (mean 4%, n=10). Inter-assay CVs were <10% (mean 6%) and <13% (mean 6%) for native insulin and ROS-INS (n=12), respectively.

**Table 5: Clinical and biochemical features of the studied populations. Gender, age and metabolic parameters of type 1 diabetes mellitus (T1D), type 2 diabetes mellitus (T2D) and healthy controls. Data are mean ± standard error.**

| **Characteristic** | **Type 1 diabetes** | | **Type 2 diabetes (*n*=64)** | **No diabetes** | |
|---|---|---|---|---|---|
| | **At diagnosis (*n*=116)** | **After insulin (*n*=69)** | | **Children (*n*=45)** | **Adults (*n*=68)** |
| Age, years | 13.9±0.8 | 14.8±1.0 | 59.5±3.4 | 14.2±0.6 | 38.0±1.4 |
| Ratio of male:female | 0.85 | 0.97 | 1 | 0.33 | 0.66 |
| Blood glucose, mmol/l | 21.4±0.7 | 8.4±0.7 | 8.6±0.4 | 4.2±0.1 | |
| HbA_{1c}, % (mmol/mol) | 11.0±0.3 (96±2.9) | 6.8±0.3 (51±3.1) | 7.4±0.3 (57±2.9) | 5.4±0.1 (39±4.02) | |
| BMI, kg/m² | 18.7±0.4 | 18.7±0.5 | 31.8±2.1 | 21.4±0.8 | |

A comparison between ROS-INS ELISA and a radiobinding assay (RBA) was also performed. At diagnosis, reactivity to ROS-INS (^{•}OH-INS and/or HOCI-INS) and IAA in RBA was detected in 84% and 61% of participants with type 1 diabetes, respectively (Fig. 5a-b). The sensitivity of ROS-INS antibodies was 84% vs 61% for IAA measured by RBA (p=0.0001), while specificity was 99% for both assays. ROS-INS antibodies by ELISA and IAA by RBA coexisted in 50% of individuals with type 1 diabetes, but ROS-INS antibodies were able to detect an additional 34% of participants who were IAA negative. Overall, 82.7% of participants who were positive on RBA were also ROS-INS positive on ELISA, while 17.3% who were positive on RBA were ROS negative on ELISA. The combined measurement of IAA by RBA and ROS-INS antibody ELISA raised the detection of insulin autoimmunity to 95% of participants with new-onset type 1 diabetes.

### Example 5 - Antibody binding specificity to ROS by competitive ELISA and western blot

A competitive displacement assay was performed to evaluate serum binding specificities to ROS-INS by pre-incubating sera with either native insulin or ROS-INS (Fig. 6). When type 1 diabetes sera were pre-incubated with an excess amount of native insulin, no significant displacement occurred and the competitive assay showed only a 25% lower absorbance compared with the direct binding ROS-INS assay. In contrast, pre-incubation of sera with an excess of ROS-INS (^{•}OH-INS or HOCI-INS) led to a strong reduction in binding (88% mean reduction in absorbance), indicating that antibody specificities are mainly to ROS-INS. Western blot analysis was then performed on a range of serum samples that exhibited the strongest binding in ELISAs. Weak binding to a fragment corresponding to native insulin was evident, but this was lost after glycation. Binding to ^{•}OH -INS and HOCI-INS was stronger and directed towards a diffused fragment that had slower mobility than native insulin. Serum samples from healthy controls did not bind to any of the insulin formats (data not shown).

The relationship of antibody binding to ROS-insulin with clinical features was also assessed. Binding of samples from individuals with type 1 diabetes to native insulin and ROS-INS was unrelated to indices of metabolic control, such as fasting plasma glucose and HbA1c, either at diagnosis or after metabolic compensation (-0.03<ρ<0.16, ρ>0.132), or to fasting C-peptide, insulin requirements or age (-0.07<ρ<-0.01, p>0.916), see Table 5.

### Materials and methods

In vitro chemical modifications: Human recombinant Insulin (1 mg/ml) in PBS was incubated overnight at 37°C with the following systems: (1) 2 mol/l ribose (Sigma, Gillingham, UK); (2) 9 mmol/l HOCI (BDH, Oxford, UK); (3) 4.5 mmol/l CuCl2 (Sigma) and 9 mmol/l H₂O₂ (Sigma) which was used to produce hydroxyl radical (^{•}OH) by the Fenton reaction. Hen Egg Lysozyme (HEL; Sigma) was also modified as above and used as control antigen. Modification of Insulin was monitored by 20% reducing sodium dodecyl sulfate-polyacrylamide gel electrophoresis (SDS-PAGE) as well as 20% native PAGE followed by staining with Coomassie (Sigma).

Three dimensional (3-D) fluorescence: 3-D scanning fluorescence spectra were obtained using a Hitachi F-4500 spectrofluorometer (Tokyo, Japan). Samples were briefly centrifuged prior to scanning, to remove aggregated material. Simultaneous excitation (200-800 nm) and emission (200-800 nm) spectra were recorded.

Mass spectrometry: Samples were desalted with StageTipC18 and analyzed by MALDI-TOF in positive, linear mode, mass range m/z from 2,000 to 25,000. An aliquot of the samples was reduced with DTT, digested with Glu-C (Calbiochem, Merck, Darmstadt, Germany), desalted by StageTipC18 and submitted to MALDI-TOF (MS) and MALDI-TOF-TOF (MS/MS) analysis and nano-electrospray ionization tandem mass spectrometry (nano-ESI-MS/MS).

Serum samples: Serum samples were obtained from participating centres of the Immunotherapy Diabetes (IMDIAB) group. Individuals with type 1 diabetes (T1D) were diagnosed according to American Diabetes Association criteria-in most cases diagnosis was confirmed by positivity to glutamic acid decarboxylase, insulin or tyrosine phosphatase antibodies; patients with other major chronic disease were excluded. T1D samples were collected at diagnosis, before insulin therapy was started. Sera from individuals with type 2 diabetes (n=82) and healthy individuals (n=68) were used as control groups. This project was approved by the Ethical Committee at University Campus Bio-Medico within the framework of the IMDIAB investigators type 1 diabetic study, with informed consent signed by patients or parents.

ELISA: An ELISA was performed using modified and native insulin or HEL as targets. Briefly, ELISA plates (Nunc, London, UK) were coated with 10 µg/ml of modified or native protein in 0.05M Carbonate/Bicarbonate Buffer pH 9.6 at 4° C overnight. Plates were then washed three times with PBS. After blocking for 2 h with 5% BSA in 0.5% Tween PBS, 100 µl of 1:200-diluted serum samples in 5% BSA in 0.5% Tween PBS were added to each well, followed by 2 h incubation at room temperature. Plates were then washed with PBS plus 0.1% Tween, followed by three washes with PBS. Anti-human IgG-horseradish peroxidase (HRP)-conjugated antibodies (Sigma) were then added at 1:1,000 dilution in 5% BSA in 0.5% Tween PBS for another 2 h incubation. The ELISA plates were washed, and 100 µg/ml 3,3',5,5'-tetramethylbenzidine substrate (Sigma) in 100 mmol/l sodium acetate, pH6.0, were added. Subsequently, the reaction was stopped with 1 mol/l sulphuric acid. The optical density (OD) was measured at 450 nm using a GENios plate reader and Magellan software (Tecan, Reading, UK). The ELISA absorbance values obtained for HEL and oxPTM-HEL were used as background controls that were subtracted from the absorbance values of native insulin and oxPTM-INS, respectively. In addition, to control assay fluctuation, binding to insulin, oxPTM-INS, HEL and oxPTM-HEL was tested for each individual sample on the same plate. Each assay included known positive or negative reference control samples. Longitudinal samples obtained from the same individuals (before and after insulin treatment) were tested on the same plate. Levels of oxPTM-INS antibodies above the 99th percentile of the healthy individuals were defined as the ELISA cut-off. A competitive ELISA was performed to assess the binding specificity of serum to oxPTM-INS. The competitive ELISA was carried out in a similar manner as above, except that the serum samples were pre-incubated for 2 h or overnight, with and without 10 µg/ml native insulin or oxPTM-INS as the competitor, before adding the serum samples to the coated ELISA plate.

Radiobinding assay (RBA): IAAs were measured by RBA using a modified radioimmunoprecipitation assay. Briefly, 20 µl serum was incubated for 2 days at 4°C in the presence of human 125-I insulin. Immune complexes were then precipitated by using 50% protein A/G-Sepharose. After several washings, bound 125-I insulin was measured in a beta counter. Results were expressed as an index defined as follows: (sample cpm - negative standard control cpm) / (positive standard control cpm - negative standard control cpm). Serum samples with an index >0.002 were considered to be positive. The assay limit of positivity was calculated according to >99th percentile values of 150 healthy control sera. This assay achieved 46% sensitivity and 100% specificity at the Islet Autoantibody Standardization Proficiency Workshop in 2012.

Western blot: PAGE was run as described above and transfer to nitrocellulose by standard protocols. Membrane was blocked in 5% dry milk powder (Marvel) for 2h followed by overnight incubation at 4°C with 1:1000 diluted serum sample in 5% marvel. After washing twice for five minutes in 0.5% Tween-PBS and five minutes in PBS, membrane was incubated for 1 hour with anti-IgG HRP (Sigma) 1:1000 in 5% marvel. Membrane was then washed twice for ten minutes in 0.5% Tween-PBS and ten minutes in PBS before incubating with 1 ml of Luminata Forte Western HRP (Sigma). Bands were detected using FluorChem E System (Proteinsimple, Santa Clara, California, USA).

HLA genotyping: HLA typing was performed by PCR using specific primers and hybridization with sequence specific oligonucleotides, as previously described in Strollo et al (Diabetologia Mar 2013).

Statistical analysis: Statistical analyses were performed using Prism Software (GraphPad, San Diego, CA, USA) and SPSS 19 (IBM, USA). Differences in levels of auto-antibodies between groups were tested by the Mann-Whitney test. Longitudinal changes in antibody binding were evaluated in 54 patients with T1D by the paired t-test, or by Wilcoxon test paired if data were not normally distributed. Levels of anti-native and ROS-INS and HEL antibodies above the 97.5th percentile of the healthy individuals were defined as elevated. Pearson's correlation test was used to assess the association between reactivity to ROS-INS and native-INS and metabolic control. Odds ratios (OR) were calculated and reported with 95% CIs to assess the association between genotype and presence of elevated insulin auto-antibodies.

## Claims

1. A method of diagnosing type 1 diabetes (T1D) in a subject comprising:
testing a sample from the subject for the presence or absence of antibodies against oxidative post-translationally modified insulin;
wherein the presence of antibodies against oxidative post-translationally modified insulin in the sample is indicative of T1D in the subject.

2. A method according to claim 1 wherein the modified insulin has been modified by non-enzymatic glycation or a reactive oxygen species (ROS).

3. A method according to claim 2 wherein the ROS is selected from the group consisting of superoxide radical (O₂^{•-}), hydrogen peroxide (H₂O₂), hydroxyl radical (^{•}OH), hypochlorous acid (HOCI), nitric oxide (NO^{•}) and peroxynitrite (ONOO⁻).

4. A method according to any one of claims 1 to 3, wherein the oxidative post-translationally modified insulin is a fragment of oxidative post-translationally modified insulin.

5. A method according to any one of the preceding claims, wherein insulin chain B is oxidative post-translationally modified at one or more of residues 21-30.

6. A method according to claim 5, wherein Phe24 and/or Tyr26 of insulin chain B is oxidised.

7. A method according to any one of the preceding claims wherein the sample is blood or serum.

8. A method according to any one of the preceding claims wherein the presence or absence of antibodies against modified insulin is determined using an ELISA assay.

9. A method according to any one of claims 1 to 3, 7 and 8 wherein the subject has not been diagnosed with diabetes.

10. A method according to any one of the preceding claims wherein the T1D is latent autoimmune diabetes in adults (LADA).

11. A method according to any one of the preceding claims wherein the subject has no insulin autoantibodies (IAA), islet cell antibodies (ICA), glutamic acid decarboxylase autoantibodies (GADA), insulinoma-associated-2 autoantibodies (IA-2A) and/or autoantibodies to ZnT8A.

12. Insulin for use in a method of treating T1D in a subject in need thereof, wherein the method comprises:
testing a sample from the subject for the presence or absence of antibodies against oxidative post-translationally modified insulin;
identifying the presence of antibodies against oxidative post-translationally modified insulin in the sample; and administering insulin to the subject having presence of antibodies against oxidative post-translationally modified insulin.

13. Insulin for use according to claim 12 wherein the T1D is LADA.

14. Use of a kit in a method of diagnosing T1D in a subject according to claim 1, wherein the kit comprises reagents for determining the presence of antibodies against oxidative post-translationally modified insulin in a sample from a subject.

15. Use according to claim 14, wherein the T1D is LADA.

## Patentansprüche

1. Verfahren zur Diagnose von Typ-1-Diabetes (T1D) in einem Subjekt, umfassend:
Testen einer Probe aus dem Subjekt auf Anwesenheit oder Abwesenheit von Antikörpern gegen oxidatives post-translational modifiziertes Insulin;
wobei die Anwesenheit von Antikörpern gegen oxidatives posttranslational modifiziertes Insulin in der Probe auf T1D in dem Subjekt hinweist.

2. Verfahren gemäß Anspruch 1, wobei das modifizierte Insulin durch nichtenzymatische Glykierung oder eine reaktive Sauerstoffspezies (ROS) modifiziert worden ist.

3. Verfahren gemäß Anspruch 2, wobei die ROS ausgewählt ist aus der Gruppe bestehend aus Superoxidradikal (O₂^{•-}), Wasserstoffperoxid (H₂O₂), Hydroxylradikal (^{•}OH), Hypochlorsäure (HOCl), Stickoxid (NO^{•}) und Peroxynitrit (ONOO⁻).

4. Verfahren gemäß einem der Ansprüche 1 bis 3, wobei das oxidative posttranslational modifizierte Insulin ein Fragment von oxidativem posttranslational modifiziertem Insulin ist.

5. Verfahren gemäß einem der vorhergehenden Ansprüche, wobei Insulinkette B an einem oder mehreren der Reste 21-30 oxidativ posttranslational modifiziert ist.

6. Verfahren gemäß Anspruch 5, wobei Phe24 und/oder Tyr26 von Insulinkette B oxidiert ist.

7. Verfahren gemäß einem der vorhergehenden Ansprüche, wobei die Probe Blut oder Serum ist.

8. Verfahren gemäß einem der vorhergehenden Ansprüche, wobei die Anwesenheit oder Abwesenheit von Antikörpern gegen modifiziertes Insulin unter Verwendung eines ELISA-Assays bestimmt wird.

9. Verfahren gemäß einem der Ansprüche 1 bis 3, 7 und 8, wobei bei dem Subjekt kein Diabetes diagnostiziert wurde.

10. Verfahren gemäß einem der vorhergehenden Ansprüche, wobei es sich bei dem T1D um latenten Autoimmundiabetes bei Erwachsenen (LADA) handelt.

11. Verfahren gemäß einem der vorhergehenden Ansprüche, wobei das Subjekt keine Insulin-Autoantikörper (IAA), Inselzell-Antikörper (ICA), Glutaminsäuredecarboxylase-Autoantikörper (GADA), Insulinom-assoziierte-2-Autoantikörper (IA-2A) und/oder Autoantikörper gegen ZnT8A aufweist.

12. Insulin zur Verwendung in einem Verfahren zur Behandlung von T1D bei einem Subjekt, das dessen bedarf, wobei das Verfahren umfasst:
Testen einer Probe aus dem Subjekt auf Anwesenheit oder Abwesenheit von Antikörpern gegen oxidatives post-translational modifiziertes Insulin;
Identifizieren der Anwesenheit von Antikörpern gegen oxidatives posttranslational modifiziertes Insulin in der Probe; und Verabreichen von Insulin an das Subjekt, das eine Anwesenheit von Antikörpern gegen oxidatives posttranslational modifiziertes Insulin aufweist.

13. Insulin zur Verwendung gemäß Anspruch 12, wobei es sich bei dem T1D um LADA handelt.

14. Verwendung eines Kits in einem Verfahren zur Diagnose von T1D in einem Subjekt gemäß Anspruch 1, wobei das Kit Reagenzien zum Bestimmen der Anwesenheit von Antikörpern gegen oxidatives posttranslational modifiziertes Insulin in einer Probe aus einem Subjekt umfasst.

15. Verwendung gemäß Anspruch 14, wobei es sich bei dem T1D um LADA handelt.

## Revendications

1. Procédé de diagnostic du diabète de type 1 (DT1) chez un sujet, comprenant :
l'analyse d'un échantillon provenant du sujet pour détecter la présence ou l'absence d'anticorps contre une insuline ayant subi une modification oxydative post-traductionnelle ;
dans lequel la présence d'anticorps contre l'insuline ayant subi une modification oxydative post-traductionnelle dans l'échantillon indique la présence d'un DT1 chez le sujet.

2. Procédé selon la revendication 1, dans lequel l'insuline modifiée a été modifiée par glycation non enzymatique ou une espèce réactive de l'oxygène (ERO).

3. Procédé selon la revendication 2, dans lequel l'ERO est sélectionnée dans le groupe constitué d'un radical superoxyde (O₂^{•-}), du peroxyde d'hydrogène (H₂O₂), d'un radical hydroxyle (^{•}OH), de l'acide hypochloreux (HOCl), de l'oxyde nitrique (NO^{•}) et du peroxynitrite (ONOO⁻).

4. Procédé selon l'une quelconque des revendications 1 à 3, dans lequel l'insuline ayant subi une modification oxydative post-traductionnelle est un fragment d'insuline ayant subi une modification oxydative post-traductionnelle.

5. Procédé selon l'une quelconque des revendications précédentes, la chaîne B d'insuline étant modifiée de manière oxydative post-traductionnelle au niveau d'un ou plusieurs des résidus 21 à 30.

6. Procédé selon la revendication 5, Phe24 et/ou Tyr26 de la chaîne B d'insuline étant oxydé(s).

7. Procédé selon l'une quelconque des revendications précédentes, dans lequel l'échantillon est du sang ou du sérum.

8. Procédé selon l'une quelconque des revendications précédentes, dans lequel la présence ou l'absence d'anticorps contre l'insuline modifiée est déterminée en utilisant un test ELISA.

9. Procédé selon l'une quelconque des revendications 1 à 3, 7 et 8, dans lequel le sujet n'a pas reçu de diagnostic de diabète.

10. Procédé selon l'une quelconque des revendications précédentes, dans lequel le DT1 est le diabète auto-immun latent de l'adulte (LADA).

11. Procédé selon l'une quelconque des revendications précédentes, dans lequel le sujet ne possède pas d'auto-anticorps anti-insuline (IAA), d'anticorps anti-îlots de Langerhans (ICA), d'auto-anticorps anti-acide glutamique décarboxylase (GADA), d'auto-anticorps anti-IA-2 associés à un insulinome (IA-2A) et/ou d'auto-anticorps anti-ZnT8 (ZnT8A).

12. Insuline destinée à une utilisation dans un procédé de traitement d'un DT1 chez un sujet ayant besoin d'un tel traitement, le procédé comprenant :
l'analyse d'un échantillon provenant du sujet pour détecter la présence ou l'absence d'anticorps contre une insuline ayant subi une modification oxydative post-traductionnelle ;
l'identification de la présence d'anticorps contre l'insuline ayant subi une modification oxydative post-traductionnelle dans l'échantillon ; et l'administration d'insuline au sujet possédant des anticorps contre l'insuline ayant subi une modification oxydative post-traductionnelle.

13. Insuline destinée à une utilisation selon la revendication 12, le DT1 étant le LADA.

14. Utilisation d'une trousse dans un procédé de diagnostic du DT1 chez un sujet selon la revendication 1, la trousse comprenant des réactifs pour déterminer la présence d'anticorps contre l'insuline ayant subi une modification oxydative post-traductionnelle dans un échantillon provenant d'un sujet.

15. Utilisation selon la revendication 14, le DT1 étant le LADA.
